(19) Europäisches Patentamt
European Patent Office
Office européen des brevets

(11) **EP 1 482 519 B1**

(12) **EUROPÄISCHE PATENTSCHRIFT**

(45) Veröffentlichungstag und Bekanntmachung des Hinweises auf die Patenterteilung:
**29.12.2010 Patentblatt 2010/52**

(21) Anmeldenummer: **04008939.3**

(22) Anmeldetag: **15.04.2004**

(51) Int Cl.:
**G21K 1/10** (2006.01)   **G21K 1/02** (2006.01)
**H01J 49/46** (2006.01)   **A61N 5/10** (2006.01)

(54) **Energiefiltereinrichtung**

Energy filtering device

Dispositif de filtrage en énergie

(84) Benannte Vertragsstaaten:
**AT BE BG CH CY CZ DE DK EE ES FI FR GB GR HU IE IT LI LU MC NL PL PT RO SE SI SK TR**

(30) Priorität: **26.05.2003 DE 10323654**

(43) Veröffentlichungstag der Anmeldung:
**01.12.2004 Patentblatt 2004/49**

(73) Patentinhaber: **GSI Helmholtzzentrum für Schwerionenforschung GmbH
64291 Darmstadt (DE)**

(72) Erfinder:
• **Kraft, Gerhard, Prof. Dr.
64291 Darmstadt (DE)**
• **Weber, Ulrich, Dr.
61352 Bad Homburg (DE)**
• **Kraft, Sebastian
72070 Tübingen (DE)**
• **Kraft, Stephan
69221 Dossenheim (DE)**

(74) Vertreter: **Mergel, Volker
Blumbach - Zinngrebe
Patentanwälte
Alexandrastrasse 5
65187 Wiesbaden (DE)**

(56) Entgegenhaltungen:
**WO-A-00/49624        US-A- 5 440 133
US-A1- 2002 162 774**

• **PATENT ABSTRACTS OF JAPAN Bd. 018, Nr. 476 (C-1246), 6. September 1994 (1994-09-06) -& JP 06 154351 A (TSUKUBA UNIV), 3. Juni 1994 (1994-06-03)**
• **GOODFELLOW: "Polymethylmethacrylat - page from german Catalogue" 1. September 2001 (2001-09-01), GOODFELLOW , BAD NAUHEIM, GERMANY , XP002397156 * das ganze Dokument ***
• **FOURKAL E ET AL: "Particle in cell simulation of laser-accelerated proton beams for radiation therapy" MEDICAL PHYSICS, AIP, MELVILLE, NY, US, Bd. 29, Nr. 12, Dezember 2002 (2002-12), Seiten 2788-2798, XP012011681 ISSN: 0094-2405**
• **PATENT ABSTRACTS OF JAPAN Bd. 1999, Nr. 05, 31. Mai 1999 (1999-05-31) -& JP 11 028252 A (MITSUBISHI ELECTRIC CORP), 2. Februar 1999 (1999-02-02)**
• **GUSTAFSSON A ET AL: "A GENERALIZED PENCIL BEAM ALGORITHM FOR OPTIMIZATION OF RADIATION THERAPY" MEDICAL PHYSICS, AIP, MELVILLE, NY, US, Bd. 21, Nr. 3, März 1994 (1994-03), Seiten 343-356, XP000435143 ISSN: 0094-2405**
• **JONES W P ET AL: "Design of a beam transport system for a proton radiation therapy facility" PARTICLE ACCELERATOR CONFERENCE, 1999. PROCEEDINGS OF THE 1999 NEW YORK, NY, USA 27 MARCH-2 APRIL 1999, PISCATAWAY, NJ, USA,IEEE, US, Bd. 4, 27. März 1999 (1999-03-27), Seiten 2519-2521, XP010349299 ISBN: 0-7803-5573-3**

**Beschreibung**

Die Erfindung betrifft Anordnungen zur Erzeugung eines Ionenstrahls mit einer Energiefiltereinrichtung

Ionenstrahlen, das heißt Protonen und schwerere Ionen, werden zur Strahltherapie von Tumorgeweben eingesetzt. Strahlen dieser Art haben gegenüber Photonen den Vorteil, dass sie ein wesentlich günstigeres Tiefendosisprofil aufweisen. Während bei Photonenbestrahlung die Dosis mit wachsender Eindringtiefe abnimmt, steigt sie bei Ionen langsam an und fällt nach einem scharfen Maximum, dem so genannten Bragg-Peak, steil ab. Die Position dieses Maximums muss genau über das Zielvolumen verteilt werden, um die Dosis auf den Tumor zu konzentrieren und gleichzeitig die Integraldosis im gesunden Gewebe zu verringern (siehe zum Beispiel die WO 00/49624 A). Dies gilt auch bei laserinduzierten Ionenstrahlen.

In jüngster Zeit wurde gezeigt, dass durch hochintensive, gepulste Laserstrahlen aus Folien relativ eng gebündelte Protonenstrahlen erzeugt werden können. Es zeigt sich jedoch, dass die laserinduzierte Strahlung ein für die Krebstherapie sehr ungünstiges Energiespektrum und damit ein ungünstiges Tiefendosisprofil aufweist.

Das Energiespektrum kann dadurch verbessert werden, dass mittels eines Magnetspektrometers ein schmales Energieband ausgeblendet und der Rest des Spektrums vernichtet wird (siehe dazu Hermann Wollnik: Optic of charged particles", Academic Express 1987 San Diego, California, USA, pages 253 to 277 (Desgin of particle spectrometers) oder auch Fourkal et al., "Particle in cell simulation of laser-accelerated proton beams for radiation therapy", Med. Phys. 29 (12), Dezember 2002, Seiten 2788 bis 2798). Bei dieser Selektionsmethode wird jedoch der weitaus größte Teil der erzeugten Protonen, nämlich mehr als 95%, ungenutzt herausgefiltert und vernichtet.

Mögliche Ionenstrahltransportsysteme für die Ionentherapie sind zum Beispiel gezeigt in der Publikation von W.P. Jones et al. ("Design of a beam transport system for proton radiation therapy facility", Proceedings of the 1999 Particle Accelerator Conference, New York, 1999, pp. 2519-2521), in dem Dokument JP 06154351 A oder in dem Dokument US-A-5 440 133. Die beiden zuletzt genannten Dokumente zielen auf eine Vergrößerung des Strahlquerschnitts ab.

Aufgabe der Erfindung ist es daher, eine Energiefiltereinrichtung zur Verfügung zu stellen, mittels derer das Energiespektrum von laserinduzierten Strahlen besser verwertet werden kann, wobei weniger Protonen ungenutzt herausgefiltert werden als bei herkömmlichen magnetischen Filtern.

Zur Lösung dieser Aufgabe wird eine Anordnung zur Erzeugung eines Ionenstrahls für die Ionenstrahltherapie umfassend eine Energiefiltereinrichtung vorgeschlagen. Die Anordnung weist die im Anspruch 1 oder im Anspruch 12 genannten Merkmale auf. Sie zeichnet sich allgemein dadurch aus, dass mindestens ein passiver Modulator vorgesehen ist. Mit diesem ist es möglich, bei einfachem Aufbau der Einrichtung das Energiespektrum der Strahlung so zu verändern, dass das Energieprofil zu höheren Energien verschoben wird, also aufgehärtet wird, wobei ein günstiges auch als Reichweitenprofil bezeichnetes Tiefendosisprofil erzeugt wird.

Die Erfindung gemäß Anspruch 1 zeichnet sich allgemein dadurch aus, dass der Modulator ein magnetisches Filter und einen Absorber aufweist.

Anspruch 1 beschreibt eine Anordnung zur Erzeugung eines Ionenstrahls für die Ionenstrahltherapie umfassend

- einen Laser zur Erzeugung eines Laserstrahls und
- ein Target, wobei der Laserstrahl auf das Target auftrifft, so dass ein breit gestreuter Ionenstrahl mit einem breiten Energiespektrum erzeugt wird, umfassend
- einen ersten Kollimator, welcher dem Target nachgeordnet ist, mit einer Kollimatoröffnung, durch die ein Teil des breit gestreuten Ionenstrahls tritt, so dass sich ein kollimierter Strahl ergibt und
- eine Energiefiltereinrichtung mit mindestens einem passiven Modulator, der Aufhärtung des Strahls dienend, so dass das Energiespektrum dN/dE des Strahls durch den passiven Modulator so verändert ist, dass das Energieprofil des Strahls zu höheren Energien verschoben ist

wobei der Modulator folgendes aufweist:

ein magnetisches Filter zur Erzeugung eines ersten Magnetfeldes, so dass sich ein spektral gespreizter Strahl ergibt, der räumlich nach der Energie der Teilchen separiert ist, einen Absorber, der Energie-Homogenisierung des gespreizten Strahls dienend, und einen zweiten Kollimator, durch den eine obere und eine untere Energiegrenze des gespreizten Strahls, der den Absorber durchlaufen hat, festgelegt sind.

Die Erfindung gemäß Anspruch 13 zeichnet sich allgemein dadurch aus, dass der Modulator ein nicht-lineares Filter aufweist, mit dem das Energieprofil und das Tiefendosisprofil eingestellt werden können.

Anspruch 12 beschreibt eine Anordnung zur Erzeugung eines Ionenstrahls für die Ionenstrahltherapie umfassend

- einen Laser zur Erzeugung eines Laserstrahls und
- ein Target, wobei der Laserstrahl auf das Target auftrifft, so dass ein breit gestreuter Ionenstrahl mit einem breiten Energiespektrum erzeugt wird, umfassend
- einen ersten Kollimator, welcher dem Target nachgeordnet ist, mit einer Kollimatoröffnung, durch die ein Teil des breit gestreuten Ionenstrahls tritt, so dass sich ein kollimierter Strahl ergibt und

- eine Energiefiltereinrichtung mit mindestens einem passiven Modulator, der Aufhärtung des Strahls dienend, so dass das Energiespektrum dN/dE des Strahls durch den passiven Modulator so verändert ist, dass das Energieprofil des Strahls zu höheren Energien verschoben ist, wobei der Modulator ein nicht-lineares Filter aufweist, das ein

erstes magnetisches Filter zur räumlichen Separation des Strahls nach der Energie der Teilchen, welches ein erstes Magnetfeld aufweist, und

eine Vorrichtung zur Beschneidung der Intensität der einzelnen Energien umfasst, so dass die räumlich nach der Energie der Teilchen separierten Strahlanteile unterschiedlich stark beschnitten sind, wobei die Vorrichtung zur Beschneidung der Intensität der einzelnen Energien einen selektiven Kollimator mit einer verjüngenden Kollimatoröffnung aufweist, und wobei die verjüngende Kollimatoröffnung des selektiven Kollimators eine Durchlassbreite besitzt, die größer für die schwach abgelenkten Strahlanteile mit höherer Energie ist als die für die stärker abgelenkten niederenergetischen Strahlanteile.

Weitere Ausgestaltungen ergeben sich aus den jeweiligen Unteransprüchen.

Ein Beispiel einer Energiefiltereinrichtung, welche nicht Gegenstand der vorliegenden Erfindung ist, zeichnet sich dadurch aus, dass der Modulator eine Streufolie und einen in einem Abstand dazu angeordneten Kollimator aufweist. Die Verwendung eines passiven Modulators dieser Art führt dazu, dass der niederenergetische Teil der Strahlung gegenüber dem Primärspektrum nachhaltig unterdrückt und das Reichweitenprofil deutlich flacher wird.

Die Erfindung wird im Folgenden anhand der Zeichnungen näher erläutert. Es zeigen:

Figur 1      eine schematische Darstellung einer nicht zur Erfindung gehörigen Energiefiltereinrichtung;

Figur 1a     das Energiespektrum eines Ionen-/Protonenstrahls an verschiedenen Positionen der nicht zur Erfindung gehörigen Energiefiltereinrichtung;

Figur 1b     das Tiefendosisprofil des Strahls an verschiedenen Stellen der nicht zur Erfindung gehörigen Energiefiltereinrichtung;

Figur 1c     die Aufstreuung für den hochenergetischen und den niederenergetischen Anteil des Strahls in der nicht zur Erfindung gehörigen Energiefiltereinrichtung;

Figur 2      eine Prinzipskizze eines Ausführungsbeispiels einer Energiefiltereinrichtung;

Figur 2a     das Energiespektrum eines Ionen-/Protonenstrahls an verschiedenen Positionen der Energiefiltereinrichtung;

Figur 2b     das Tiefendosisprofil des Strahls an verschiedenen Stellen der Energiefiltereinrichtung;

Figur 3      eine Prinzipskizze eines weiteren Ausführungsbeispiels einer Energiefiltereinrichtung und

Figur 4      einen Multi-Leaf-Kollimator in Strahlenrichtung gesehen.

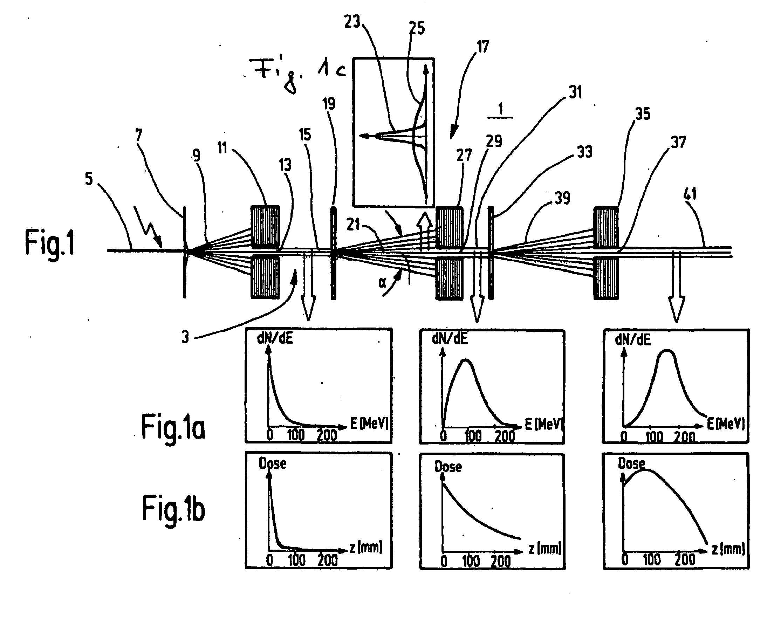

Die Figuren 1 und 1a bis 1c zeigen ein Beispiel, welches nicht Bestandteil der vorliegenden Erfindung ist.

Figur 1 zeigt eine im Folgenden kurz als Energiefiltereinrichtung 1 bezeichnete Ionenstrahlen-Energiefiltereinrichtung, die von einem Ionenstrahl 3 durchlaufen wird. Dieser wird durch einen hochintensiven, gepulsten Laserstrahl 5 erzeugt, der auf ein Target, hier auf eine Konverterfolie 7 auftrifft. Aus dieser treten schwere geladene Teilchen 9, Protonen und schwerere Ionen, aus. Das Energiespektrum der diese Teilchen 9 umfassenden Ionenstrahlung ist für eine Strahlentherapie praktisch unbrauchbar, auch das damit verbundene Reichweitenprofil.

In einem Abstand zur Konverterfolie 7 ist ein erster Kollimator 11 mit einer Kollimatoröffnung 13 angeordnet, durch die ein kollimierter Strahl 15 mit einem breiten Spektrum und einem starken niederfrequenten Anteil austritt. Der äußere Teil der Strahlung wird durch den Kollimator 11 ausgeblendet.

Unterhalb des Strahls 15 ist in Figur 1a schematisch dessen Energiespektrum wiedergegeben. Darunter findet sich in Figur 1b eine schematische Darstellung des Tiefendosisprofils des Strahls 15, der das in Figur 1a dargestellte Spektrum aufweist.

In Figur 1b ist die Strahlendosis über der Eindringtiefe z (Einheiten in mm) wiedergegeben. Figur 1b zeigt deutlich, dass die Strahlendosis stark mit der Eindringtiefe abnimmt.

Der kollimierte Strahl 15 trifft nun auf die dem Kollimator 11 nachgeordnete Energiefiltereinrichtung 1 und durchläuft diese gemäß Figur 1 von links nach rechts.

Die Energiefiltereinrichtung 1 weist einen passiven Modulator 17 auf, der bei diesem Beispiel als Streufilter ausgebildet ist. Er

umfasst eine erste Streufolie 19, die im Strahlenpfad beispielsweise in einem Abstand von 50 cm zum Kollimator angeordnet ist und auf die der kollimierte Strahl 15 auftrifft. Der Strahl wird durch Coulomb-Wechselwirkung aufgestreut. Um den Energieverlust gering zu halten, werden relativ dünne Streufolien von beispielsweise 5mm Dicke verwendet, die beispielsweise aus Plexiglas bestehen. Durch die Coulomb-Wechselwirkung entsteht eine Gauss-Verteilung für den Streuwinkel $\alpha$ nach der folgenden Gleichung:

$$f(\alpha) = \frac{1}{\sqrt{2\pi\sigma}} \exp - \frac{\alpha^2}{2\sigma^2}$$

wobei für die Varianz die folgende Beziehung gilt:

$$\sigma^2 \approx \frac{1}{E^2}$$

Die durch den ersten Kollimator 11 hindurchtretenden Teilchen 9, die auf die erste Streufolie 19 auftreffen, werden also durch diese aufgestreut, wobei die Aufstreuung umso größer ist, je kleiner die Energie der Teilchen 9 ist.

Figur 1c zeigt verschiedene Verteilungen für den Streuwinkel $\alpha$, wobei die erste Kurve 23 die Aufstreuung für den hochenergetischen Anteil der Teilchen 9 im gestreuten Strahl 21 wiedergibt und die zweite Kurve 25 die Aufstreuung für den niederenergetischen Teil der Teilchen.

Die Energiefiltereinrichtung 1 weist einen in einem Abstand zur ersten Streufolie 19 angeordneten zweiten Kollimator 27 mit einer Kollimatoröffnung 29 mit einem Durchmesser von beispielsweise 5 mm, durch die entsprechend der ersten Kurve 23 der Großteil des hochenergetischen Anteils der Teilchen 9 hindurchtritt und nur ein geringer Anteil der niederenergetischen Teilchen. Damit ergibt sich ein durch den Kollimator aufgehärteter Strahl 31, also ein Strahl, der ein für die Strahlentherapie wesentlich günstigeres Energiespektrum aufweist als der breit gestreute Ionen-/Protonenstrahl, der die Konverterfolie 7 verlässt.

Blendet man also durch den zweiten Kollimator 27 den äußeren Teil des gestreuten Strahls 21 nach einer Laufstrecke hinter der ersten Streufolie 19 aus, so erhält man einen Transmissionsfilter mit einer 1/E²-Charakteristik. Diese ergibt sich deshalb, weil der beispielsweise eine quadratische oder runde Kollimatoröffnung aufweisende Kollimator den Strahl in zwei Richtungen beschneidet.

Unterhalb des aufgehärteten Strahls 31 ist in Figur 1a das Energiespektrum dieses Strahls 31 dargestellt. Darunter findet sich in Figur 1b eine schematische Darstellung des Tiefendosisprofils des aufgehärteten Strahls 31 mit dem in Figur 1a dargestellten Energiespektrum.

Figur 1b zeigt deutlich, dass das Tiefendosisprofil des aufgehärteten Strahls 31 abweicht von dem des kollimierten Strahls 15: Die Strahlendosis fällt über die Eindringtiefe nicht mehr so steil ab, das Tiefendosisprofil ist also deutlich flacher als bei dem kollimierten Strahl 15.

Die in Figur 1 dargestellte Energiefiltereinrichtung 1 weist nach allem eine erste Aufhärtestufe auf, die die erste Streufolie 19 und den zweiten Kollimator 27 umfasst. Figur 1 zeigt aber auch, dass bei dem hier dargestellten Beispiel der Energiefiltereinrichtung 1 der passive Modulator 17 eine zweite Aufhärtestufe aufweist, die eine in einem Abstand zum zweiten Kollimator 27 angeordnete zweite Streufolie 33 und einen wiederum in einem Abstand zur zweiten Streufolie 33 angeordneten dritten Kollimator 35 mit einer Kollimatoröffnung 37 umfasst.

Beim Durchlaufen der zweiten Aufhärtestufe geschieht das anhand der ersten Aufhärtestufe Erläuterte entsprechend: Der aufgehärtete Strahl 31 wird beim Durchlaufen der zweiten Streufolie 33 durch Coulomb-Wechselwirkung aufgestreut. Auch hier wird, um den Energieverlust gering zu halten, ein dünner Streuer eingesetzt, so dass man wiederum eine Gauss-Verteilung für den Streuwinkel $\alpha$ erhält, wie sie oben abgehandelt wurde.

Nach der zweiten Streufolie 33 stellt sich also wiederum ein gestreuter Strahl 39 ein, innerhalb dessen die Teilchen mit einer hohen Energie einen geringeren Streuwinkel aufweisen als die niederenergetischen Teilchen, wie dies anhand von Figur 1c erläutert wurde.

Durch den der zweiten Streufolie nachgeordneten dritten Kollimator 35 wird wiederum der äußere Teil des gestreuten Strahls 39, wie bei dem gestreuten Strahl 21, ausgeblendet. Durch die Kollimatoröffnung 37 treten also wesentlich mehr hochenergetische Teilchen als niederenergetische Teilchen aus, so dass sich ein aufgehärteter Strahl 41 ergibt. Unterhalb dieses Strahls ist in Figur 1a das Energiespektrum dieses Strahls dargestellt, das hier etwa bei 200 MeV ein deutliches Maximum aufweist, und in Figur 1b das Tiefendosisprofil dieses Strahls mit dem in Figur 1a dargestellten Spektrum.

Deutlich wird aus Figur 1b, dass die effektive Dosis des Strahls mit zunehmender Eindringtiefe zunimmt und nach Erreichen eines Maximums, etwa bei einer Eindringtiefe von ca. 100 mm abfällt.

Die Energiefiltereinrichtung 1 nach Figur 1 weist zwei Aufhärtestufen auf. Denkbar ist es aber auch mehr als zwei derartiger Stufen zu verwenden.

Die Dicke der Streufolien 19, 23 und die Größe der Kollimatoröffnungen 29, 37 sowie der Abstand zwischen den Streufolien und den Kollimatoren 27, 35 sind variabel und auf das Energiespektrum des in die Energiefiltereinrichtung eintretenden Ionen-/Protonenstrahls abstimmbar.

Durch die Dicke der Streufolien 19, 33 und die Größe der Kollimatoröffnungen 29, 37 kann die Energiefiltereinrichtung 1 an den auch als Primarstrahl bezeichneten kollimierten Strahl 15 angepasst werden.

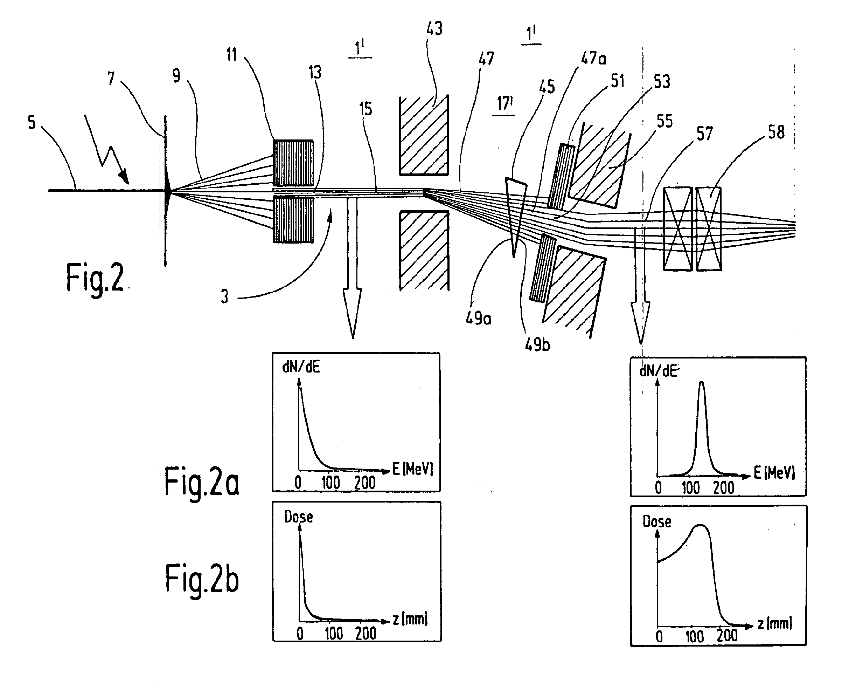

Figur 2 zeigt ein erstes Ausführungsbeispiel einer Energiefiltereinrichtung 1', die von einem Ionenstrahl 3 durchlaufen wird. Auch hier wird davon ausgegangen, dass mittels eines Laserstrahls 5, der auf ein Target, hier auf eine Konverterfolie 7 trifft, ein breit gestreuter Ionenstrahl mit einem breiten Spektrum erzeugt wird, indem Teilchen 9 (Protonen und schwere Ionen) aus der Konverterfolie 7 herausgesprengt werden.

In einem Abstand zur Konverterfolie 7 befindet sich ein erster Kollimator 11 mit einer Kollimatoröffnung 13, durch die ein Teil des breit gestreuten Protonenstrahls tritt, so dass sich ein kollimierter Strahl 15 ergibt. Unterhalb dieses Strahls ist in Figur 2a das Energiespektrum dieses Strahls dargestellt und in Figur 2b das Tiefendosisprofil dieses Strahls.

Die Produktion des Teilchenstrahls und dessen erste Kollimation stimmen überein mit den in Figur 1 dargestellten Gegebenheiten.

Die Energiefiltereinrichtung 1' weist einen passiven Modulator 17' auf, der ein magnetisches Filter 43 und einen Absorber 45 umfasst.

Das magnetische Filter 43 weist ein homogenes Magnetfeld auf und wird beispielsweise durch einen elektromagnetischen Dipol realisiert. Der kollimierte Strahl 15 verläuft durch das Magnetfeld, so dass sich ein spektral gespreizter Ionen-/Protonenstrahl 47 ergibt. In Figur 2 ist der gespreizte Strahl 47 nach unten gefächert, wobei hochenergetische Teilchen durch das Magnetfeld weniger abgelenkt werden als Teilchen mit einer niedrigeren Energie und damit einer niedrigeren Geschwindigkeit.

In den Verlauf des gespreizten Strahls 47 wird der Absorber 45 eingebracht, der keilförmig ausgebildet ist, dergestalt, dass oben der dickere Teil des Keils angeordnet ist und unten der dünnere. Damit durchlaufen die hochenergetischen Ionen/Protonen den dickeren Teil und die niederenergetischen den dünneren Teil des Absorbers. Der Absorber dient der Energie-Homogenisierung und einer damit verbundenen Transmissionserhöhung.

Der Winkel der keilförmig zueinander angeordneten Wände 49a, 49b des Absorbers 45 kann auf den gewünschten Energieverlust abgestimmt werden, der bei Durchtritt des gespreizten Strahls 47 durch den Absorber 45 gewünscht wird. Es ist auch möglich, die Wände 49a und 49b, die hier geradlinig verlaufen, also in einer Ebene liegen, konkav oder konvex gewölbt auszubilden, um die Energieabsorption gezielt zu beeinflussen.

Durch eine entsprechende Dimensionierung des Absorberkeils, also durch ein geeignetes Dickenprofil des Absorbers 45, ist es also möglich, die unterschiedlichen Energien der Teilchen im gespreizten Strahl 47 durch den unterschiedlichen Energieverlust innerhalb des Absorbers auszugleichen und damit ein stark verbessertes Reichweitenprofil zu erhalten.

Dem Absorber 45 ist ein zweiter Kollimator 51 mit einer Kollimatoröffnung 53 nachgeordnet, der dazu dient, aus dem gespreizten Strahl 47, der den Absorber 45 durchlaufen hat, einen gewünschten Energiebereich herauszufiltern.

Der durch die Kollimatoröffnung 53 austretende Strahl wird mittels eines Magneten 55 parallelisiert, indem ein zweites, dem ersten Magnetfeld entgegengesetzt gepoltes Magnetfeld aufgebaut wird.

Nach Durchlaufen den zweiten Magnetfelds ergibt sich ein Strahl 57 mit höherer Energieschärfe.

In Figur 2a ist unterhalb des gespreizten Strahls 47 dessen Energiespektrum dargestellt und in Figur 2b dessen Tiefendosisprofil. Es wird deutlich, dass das Energiespektrum einen diskreten Höhepunkt aufweist und dass dadurch das Tiefendosisprofil ein relativ scharfes Maximum, einen Bragg-Peak, aufweist.

Durch den zweiten Kollimator 51 werden die obere und die untere Energiegrenze des durch den Absorber 45 beeinflussten Strahls 47a festgelegt. Durch das Magnetfeld des Magneten 55 des passiven Modulators 17' werden die nun quasi-monoenergetischen Strahlen parallelisiert. Der das weitere Magnetfilter 45 verlassende parallelisierte Strahl 57 wird durch eine geeignete Linse, hier beispielsweise durch ein Quadrupolpaar 58 fokussiert. Damit wird ein Strahl mit höherer Energiedichte erzeugt.

Der Vorteil des keilförmigen Absorbers 45 besteht in dessen hoher Transmission. Nachteilig ist, dass das gesamte Spektrum des gespreizten Strahls 47 zu niedrigeren Energien verschoben wird. Mit einer Energiefiltereinrichtung 1' der hier beschriebenen Art kann man den Intensitätsanteil der Strahlung, der oberhalb einer gewünschten Energie liegt, ohne wesentlichen Intensitätsverlust auf eine gewünschte Energie abbremsen. Bei einer magnetischen Energieselektion ohne keilförmigen Absorber, nur durch einen Kollimator, würde dieser Intensitätsanteil verloren gehen. Ein derartiger Filter würde deshalb eine ungünstige Transmissionskurve zeigen

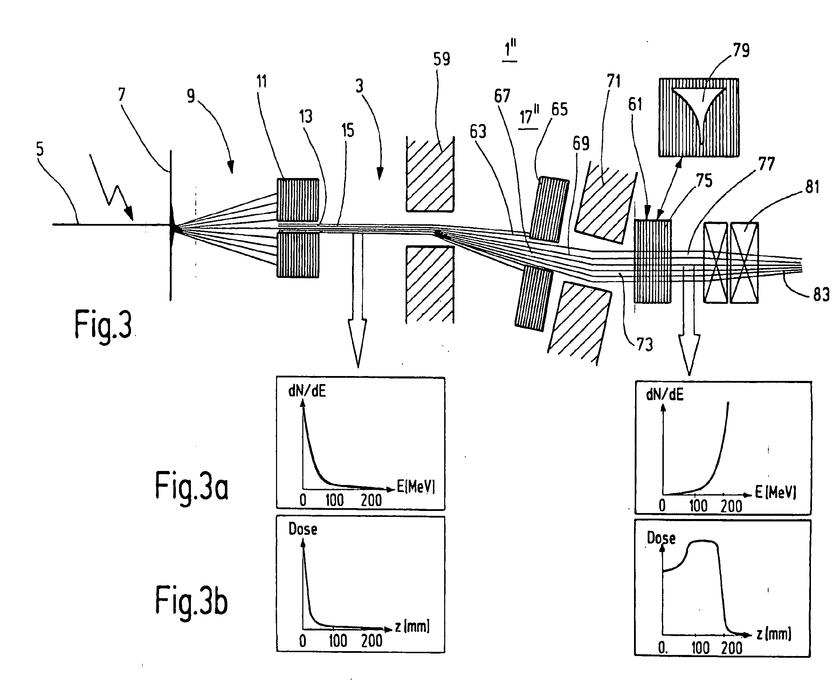

Figur 3 zeigt ein zweites Ausführungsbeispiel einer Energiefiltereinrichtung 1 ", durch die ein Ionenstrahl 3 verläuft.

Auch bei dem hier dargestellten Ausführungsbeispiel wird der Ionenstrahl mittels eines Lasers 5 hergestellt, der auf ein Target, hier auf eine Konverterfolie 7, auftrifft, so dass aus dieser geladene Teilchen 9, also Protonen und schwere Ionen, herausgesprengt werden. Der Konverterfolie 7 ist ein erster Kollimator 11 nachgeordnet, der eine Kollimatoröffnung 13 aufweist, durch die ein kollimierter Strahl 15 hindurch tritt, dessen Energiespektrum in Figur 3a und dessen Tiefendosisprofil in Figur 3b dargestellt ist.

Dieser Strahl wird der Energiefiltereinrichtung 1" zugeführt, die einen passiven Modulator 17" umfasst. Bei dem hier dargestellten Ausführungsbeispiel weist der Modulator 17" ein nicht-lineares Filter auf, das ein erstes magnetisches Filter 59 und eine Vorrichtung 61 zur Beschneidung der Intensität der einzelnen Energien des Strahls aufweist.

Das magnetische Filter 59 weist ein vorzugsweise homogenes Magnetfeld auf, das den das magnetische Filter 59 durchlaufenden kollimierten Strahl 15 spektral spreizt. Nach Figur 3 wird der kollimierte Strahl 15 nach unten aufgefächert, wobei in dem gespreizten Strahl 63, wie bei dem Strahl 47 gemäß Figur 2, Teilchen hoher Energie und damit höherer Geschwindigkeit eine geringere Ablenkung als niederenergetische Teilchen erfahren. Die geladenen Teilchen 9 werden also in dem ersten homogenen Magnetfeld des magnetischen Filters 59 nach Ihrer Energie und Geschwindigkeit separiert.

Dem magnetischen Filter 59 ist ein zweiter Kollimator 65 mit einer Kollimatoröffnung 67 nachgeordnet, der die obere und untere Energiegrenze des gespreizten Strahls 63 festlegt, so dass ein Strahl 69 erzeugt wird.

Dieser Strahl 69 wird durch einen Magneten 71 parallelisiert, wobei hier ein zweites, dem ersten Magnetfeld entgegengesetztes Magnetfeld verwendet wird und der Strahl entgegengesetzt zu der im ersten Magnetfilter 59 gegebenen Richtung abgelenkt wird.

Der aus dem Magneten 71 austretende parallelisierte Strahl 73 weist also eine höhere Energieschärfe auf als der gespreizte Strahl 63. Dieser parallelisierte Strahl 73 wird durch die Vorrichtung 61 zur Beschneidung der Intensität der einzelnen Energien, nämlich durch einen selektiven Kollimator geleitet, so dass die räumlich nach Energie separierten Strahlanteile unterschiedlich stark beschnitten werden und ein gefilterter Strahl 77 erzeugt wird.

In Figur 3 ist auch eine in Strahlrichtung gesehene Ansicht des selektiven Kollimators wiedergegeben. Deutlich wird hier die speziell ausgebildete Kollimatoröffnung 79, die in Strahlrichtung dargestellt ist und oben eine breitere freie Öffnung als unten aufweist. Die Kollimatoröffnung 79 verjüngt sich nach unten quasi keilförmig, wobei der Verlauf der Öffnung insbesondere durch einen Multi-Leaf-Kollimator 85 besonders gut beeinflussbar ist, um die gewünschte Öffnungskontur zu erhalten.

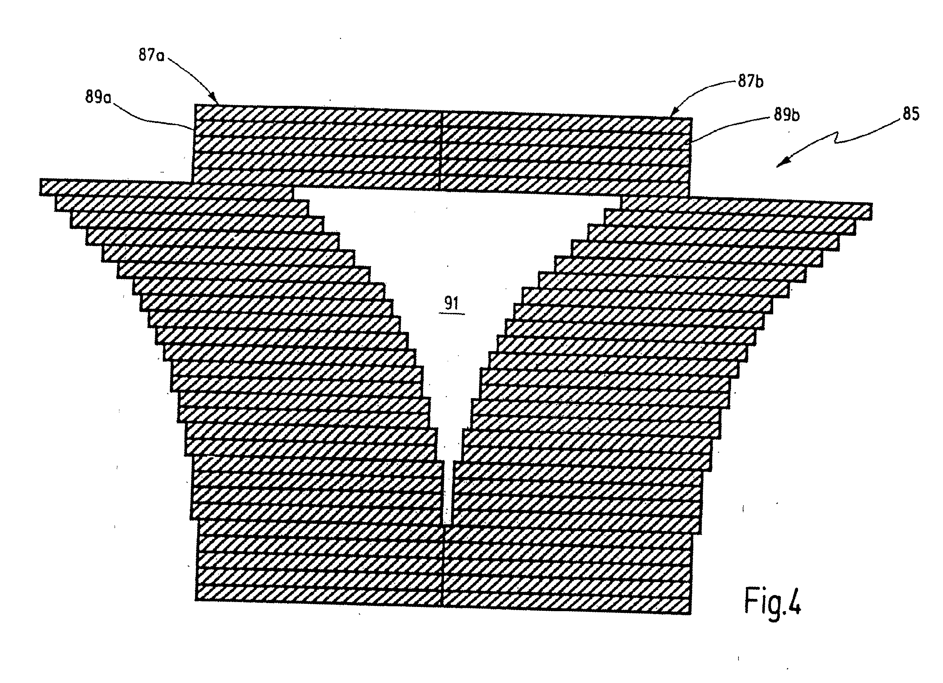

Ein Multi-Leaf-Kollimator 85 der hier angesprochenen Art ist in Figur 4 dargestellt, wobei diese Figur eine Ansicht des Kollimators in Strahlrichtung gesehen wiedergibt. Der hier dargestellte Multi-Leaf-Kollimator 85 ist vorzugsweise als Multi-Leaf-Kollimator ausgebildet und weist zwei nebeneinander liegende Gruppen 87a und 87b von paarweise angeordneten dünnen Lamellen 89a und 89b auf, die motorisch verstellt werden können.

Figur 4 zeigt, dass die obersten Lamellen der rechten und linken Gruppe 87a und 87b aufeinander zu bewegt wurden und sich mit ihren einander zugewandten Enden berühren. Unter den sich berührenden Lamellen ist eine Gruppe von Lamellen, deren einander zugewandte Enden in einem Abstand zueinander angeordnet sind, wobei dieser Abstand von oben nach unten abnimmt. Schließlich berühren sich wieder die untersten Lamellen der beiden Gruppen 87a und 87b im Bereich ihrer einander zugewandten Enden.

Die Dicke der Lamellen, von denen hier zwei einander gegenüberliegende Lamellen 89a und 89b herausgegriffen sind beträgt 2 - 4 mm. Die senkrecht zur in Figur 4 gegebenen Bildebene, also in Strahlrichtung, gemessene Lamellentiefe beträgt 5 - 10 cm, je nach dem bei der Herstellung der Lamellen verwendeten Metall.

Bei der Energiefiltereinrichtung 1" nach Figur 3 tritt der den Magneten 71 verlassende Strahl 73 durch den Multi-Leaf-Kollimator 85 hindurch. Der Strahl verläuft bei der Darstellung gemäß Figur 4 senkrecht in die hier gewählte Bildebene hinein.

Der Abstand der oberen Lamellen ist größer als der der darunter liegenden. Die Breite der Kollimatoröffnung 91 quer zur Strahlrichtung und quer zur Ablenkrichtung des Magneten 71 ist größer für den hochenergetischen Teil des Strahles, der in den Dipolen des Magneten 71 nur schwach abgelenkt wird, und wird immer kleiner für den niederenergetischen Teil des Strahls 73, der stärker abgelenkt wird. Durch die verschieblichen Lamellen 89a und 89b kann die Form der Kollimatoröffnung 91, hier die von oben nach unten gegebene Verjüngung, verändert werden, um das Energiespektrum des hinter diesem speziellen Kollimator gegebenen gefilterten Strahls 77 vorzugeben. Mit einem Multi-Leaf-Kollimator der hier angesprochenen Art kann das Spektrum des Strahls 77 individuell angepasst werden. Es ist damit möglich, ein Energiespektrum zu erzeugen, welches in einem Tiefendosisprofil mit einem Hochplateau (spread-out braggpeak) resultiert. Dies ist in den Figur 3a und 3b unterhalb des Strahls 77 wiedergegeben.

Die Erzeugung eines derartigen Strahls ist medizinisch sehr vorteilhaft.

Der gefilterte Strahl 77 kann durch eine geeignete Linse, beispielsweise durch ein Quadrupolpaar 81 geführt werden, um einen gefilterten und fokussierten Strahl 83 zu erhalten.

In Figur 3a ist das Energiespektrum des gefilterten Strahls 77 und in Figur 3b dessen Tiefendosisprofil dargestellt.

Aus den Figuren 1 bis 3 werden verschiedene Beispiele bzw. Ausführungsbeispiele von Energiefiltereinrichtungen ersichtlich. Diese weisen unterschiedliche passive Modulatoren auf. Bei den hier beschriebenen Beispiele bzw, Ausführungsbeispielen wurden jeweils ein besonderer Typ eines Modulators eingesetzt. Es ist jedoch möglich, die unterschiedlichen in den Figuren 1 bis 3 gezeigten Modulatoren und deren Elemente miteinander zu kombinieren, um eine optimale Aufhärtung des Ionenstrahls 3 zu erhalten.

Die anhand der Figuren erläuterte Energiefiltereinrichtung wird nach allem von Strahlen verwendet, die ein breites Spektrum mit einem relativ hohen niederenergetischen Anteil besitzen, wie es zum Beispiel bei der Erzeugung von Ionen durch gepulste Laserstrahlen entsteht. Es wird deutlich, dass die in den Figuren 1 bis 3 beschriebenen Modulatoren 17, 17' und 17" als Energie-Modulatoren dienen und mit dem Ziel eingesetzt werden, das Energiespektrum des Strahls aufzuhärten, um ein für die Ionenstrahlentherapie gut verwendbares Tiefendosisprofil zu erzeugen.

**Patentansprüche**

1. Anordnung zur Erzeugung eines Ionenstrahls für die Ionenstrahltherapie umfassend

   - einen Laser zur Erzeugung eines Laserstrahls (5) und
   - ein Target (7), wobei der Laserstrahl (5) auf das Target (7) auftrifft, so dass ein breit gestreuter Ionenstrahl (9) mit einem breiten Energiespektrum erzeugt wird, umfassend
   - einen ersten Kollimator (11), welcher dem Target (7) nachgeordnet ist, mit einer Kollimatoröffnung (13), durch die ein Teil des breit gestreuten Ionenstrahls (9) tritt, so dass sich ein kollimierter Strahl (15) ergibt und
   - eine Energiefiltereinrichtung (1) mit mindestens einem passiven Modulator (17') der Aufhärtung des Strahls (15) dienend, so dass das Energiespektrum dN/dE des Strahls (15) durch den passiven Modulator (17') so verändert ist, dass das Energieprofil des Strahls (15) zu höheren Energien verschoben ist

   wobei der Modulator (17') folgendes aufweist:

   ein magnetisches Filter (43) zur Erzeugung eines ersten Magnetfeldes, so dass sich ein spektral gespreizter Strahl (47) ergibt, der räumlich nach der Energie der Teilchen separiert ist, einen Absorber (45), der Energie-Homogenisierung des gespreizten Strahls (47) dienend, und einen zweiten Kollimator (51), durch den eine obere und eine untere Energiegrenze des gespreizten Strahls (47), der den Absorber (45) durchlaufen hat, festgelegt sind.

2. Anordnung nach Anspruch 1, **dadurch gekennzeichnet, dass** das Filter (43) derartig ausgestaltet ist, dass das erste Magnetfeld ein homogenes Magnetfeld ist.

3. Anordnung nach einem der vorstehenden Ansprüche, **dadurch gekennzeichnet, dass** das magnetische Filter (43) durch einen elektromagnetischen Dipol realisiert ist.

4. Anordnung nach einem der vorhergehenden Ansprüche, **dadurch gekennzeichnet, dass** der Absorber (45) ein Absorberkeil ist.

5. Anordnung nach einem der vorhergehenden Ansprüche, **dadurch gekennzeichnet, dass** der Absorber (45) eine inhomogene Dicke aufweist und keilförmig ausgebildet ist.

6. Anordnung nach einem der vorhergehenden Ansprüche, **dadurch gekennzeichnet, dass** die Wände (49a, 49b) des Absorbers (45), die einen spitzen Winkel einschließen, geradlinig verlaufen.

7. Anordnung nach einem der vorhergehenden Ansprüche 1 bis 5, **dadurch gekennzeichnet, dass** die Wände (49a, 49b) des Absorbers (45), die einen spitzen Winkel einschließen, konkav oder konvex gewölbt sind.

8. Anordnung nach einem der vorhergehenden Ansprüche, **dadurch gekennzeichnet, dass** der Absorber (45) so im Strahlverlauf des durch den magnetischen Filter (43) spektral gespreizten Strahls (47) angeordnet ist, dass Teilchen höherer Energie einen dickeren Bereich durchlaufen, als Teilchen niedrigerer Energie

9. Anordnung nach einem der vorhergehenden Ansprüche, **gekennzeichnet durch** einen Magneten (55) zur Erzeugung eines zweiten Magnetfeldes zum Parallelisieren des gespreizten Strahls.

10. Anordnung nach vorhergehendem Anspruch, **dadurch gekennzeichnet, dass** das zweite Magnetfeld des Magneten (55) umgekehrt gepolt ist wie das erste Magnetfeld des magnetischen Filters (43).

11. Anordnung nach einem der beiden vorhergehenden Ansprüche, **dadurch gekennzeichnet, dass** in Strahlrichtung vor dem zweiten Magnetfeld des Magneten (55) der zweite Kollimator (51) vorgesehen ist.

12. Anordnung zur Erzeugung eines Ionenstrahls für die Ionenstrahltherapie umfassend

    - einen Laser zur Erzeugung eines Laserstrahls (5) und
    - ein Target (7), wobei der Laserstrahl (5) auf das Target (7) auftrifft, so dass ein breit gestreuter Ionenstrahl (9) mit einem breiten Energiespektrum erzeugt wird, umfassend
    - einen ersten Kollimator (11), welcher dem Target (7) nachgeordnet ist, mit einer Kollimatoröffnung (13), durch die ein Teil des breit gestreuten Ionenstrahls (9) tritt, so dass sich ein kollimierter Strahl (15) ergibt und
    - eine Energiefiltereinrichtung (1) mit mindestens einem passiven Modulator (17"), der Aufhärtung des Strahls (15) dienend, so dass das Energiespektrum dN/dE des Strahls (15) durch den passiven Modulator (17") so verändert ist, dass das Energieprofil des Strahls (15) zu höheren Energien verschoben ist, wobei der Modulator (17") ein nicht-lineares Filter aufweist, das ein

    erstes magnetisches Filter (59) zur räumlichen Separation des Strahls nach der Energie der Teilchen, welches ein erstes Magnetfeld aufweist, und

eine Vorrichtung (61) zur Beschneidung der Intensität der einzelnen Energien umfasst, so dass die räumlich nach der Energie der Teilchen separierten Strahlanteile unterschiedlich stark beschnitten sind,

wobei die vorrichtung zur Beschneidung der Intensität der einzelnen Energien einen selektiven Kollimator (75) mit einer verjüngenden Kollimatoröffnung (79) aufweist, und

wobei die verjüngende Kollimatoröffnung (79) des selektiven Kollimators (75) eine Durchlassbreite besitzt, die größer für die schwach abgelenkten Strahlanteile mit höherer Energie ist als die für die stärker abgelenkten niederenergetischen Strahlanteile.

13. Anordnung nach Anspruch 12, **dadurch gekennzeichnet, dass** das erste magnetische Feld ein homogenes Magnetfeld ist.

14. Anordnung nach einem der vorhergehenden Ansprüche 12 bis 13, **dadurch gekennzeichnet, dass** der Modulator (17") einen Magneten (71) mit einem zweiten Magnetfeld aufweist, das umgekehrt gepolt ist wie das erste Magnetfeld des magnetischen Filters (59).

15. Anordnung nach vorhergehendem Anspruch 14 **dadurch gekennzeichnet, dass** das zweite Magnetfeld ein homogenes Magnetfeld ist.

16. Anordnung nach einem der vorhergehenden Ansprüche 14 bis 15, **dadurch gekennzeichnet, dass** dem Magneten (71) mit dem zweiten Magnetfeld ein zweiter Kollimator (65) vorgeschaltet ist.

17. Anordnung nach einem der vorgehenden Ansprüche 12 bis 16, **dadurch gekennzeichnet, dass** die Kollimatoröffnung (79) des selektiven Kollimators (75) veränderbar ist.

18. Anordnung nach einem der vorhergehenden Ansprüche 12 bis 17, **dadurch gekennzeichnet, dass** der selektive Kollimator (75) als ein Multi-Leaf-Kollimator ausgelegt ist.

**Claims**

1. Arrangement for generating an ion beam for ion beam therapy, comprising

    - a laser for generating a laser beam (5) and
    - a target (7), the laser beam (5) impinging on the target (7) so as to generate a widely scattered ion beam (9) with a broad energy spectrum, comprising

    - a first collimator (11), which is downstream of the target (7), with a collimator aperture (13) through which a portion of the widely scattered ion beam (9) passes such that a collimated beam (15) results, and
    - an energy filtering device (1) with at least one passive modulator (17') serving to harden the beam (15) such that the energy spectrum dN/dE of the beam (15) is varied by the passive modulator (17') such that the energy profile of the beam (15) is shifted toward higher energies,

    the modulator (17') having the following:

    a magnetic filter (43) for generating a first magnetic field so as to give rise to a spectrally spread beam (47) that is separated spatially by the energy of the particles,
    an absorber (45) serving to homogenize the energy of the spread beam (47), and
    a second collimator (51) which defines an upper and a lower energy limit of the spread beam (47) which has traversed the absorber (45).

2. Arrangement according to Claim 1, **characterized in that** the filter (43) is designed in such a way that the first magnetic field is a homogeneous magnetic field.

3. Arrangement according to one of the preceding claims, **characterized in that** the magnetic filter (43) is implemented by an electromagnetic dipole.

4. Arrangement according to one of the preceding claims, **characterized in that** the absorber (45) is an absorber wedge.

5. Arrangement according to one of the preceding claims, **characterized in that** the absorber (45) has an inhomogeneous thickness and is of wedge-shaped design.

6. Arrangement according to one of the preceding claims, **characterized in that** the walls (49a, 49b) of the absorber (45), which enclose an acute angle, run rectilinearly.

7. Arrangement according to one of the preceding Claims 1 to 5, **characterized in that** the walls (49a, 49b) of the absorber (45), which enclose an acute angle, are concavely or convexly cambered.

8. Arrangement according to one of the preceding claims, **characterized in that** the absorber (45) is arranged in the beam path of the beam (47) spectrally spread by the magnetic filter (43) such that particles of higher energy traverse a thicker region than do particles of lower energy.

9. Arrangement according to one of the preceding claims, **characterized by** a magnet (55) for generating a second magnetic field for the purpose of parallelizing the spread beam.

10. Arrangement according to the preceding claim, **characterized in that** the second magnetic field of the magnet (55) is of opposite polarity to the first magnetic field of the magnetic filter (43).

11. Arrangement according to one of the two preceding claims, **characterized in that** the second collimator (51) is provided upstream of the second magnetic field of the magnet (55) in the beam direction.

12. Arrangement for generating an ion beam for ion beam therapy, comprising

- a laser for generating a laser beam (5) and
- a target (7), the laser beam (5) impinging on the target (7) so as to generate a widely scattered ion beam (9) with a broad energy spectrum, comprising
- a first collimator (11), which is downstream of the target (7), with a collimator aperture (13) through which a portion of the widely scattered ion beam (9) passes such that a collimated beam (15) results, and
- an energy filtering device (1) with at least one passive modulator (17") serving to harden the beam (15) such that the energy spectrum dN/dE of the beam (15) is varied by the passive modulator (17") such that the energy profile of the beam (15) is shifted toward higher energies, the modulator (17") having a nonlinear filter that has a

first magnetic filter (59) for spatially separating the beam by the energy of the particles, which has a first magnetic field, and

comprises a device (61) for trimming the intensity of the individual energies such that the beam components separated spatially by the energy of the particles are trimmed with different intensity,

the device for trimming the intensity of the individual energies having a selective collimator (75) with a tapering collimator aperture (79), and the tapering collimator aperture (79) of the selective collimator (75) having a pass width which is larger for the weakly deflected beam components of higher energy than that for the more strongly deflected low energy beam components.

13. Arrangement according to Claim 12, **characterized in that** the first magnetic field is a homogeneous magnetic field.

14. Arrangement according to one of the preceding Claims 12 to 13, **characterized in that** the modulator (17") has a magnet (71) with a second magnetic field which is of opposite polarity to the first magnetic field of the magnetic filter (59).

15. Arrangement according to the preceding Claim 14, **characterized in that** the second magnetic field is a homogeneous magnetic field.

16. Arrangement according to one of the preceding Claims 14 to 15, **characterized in that** a second collimator (65) is connected upstream of the magnet (71) with the second magnetic field.

17. Arrangement according to one of the preceding Claims 12 to 16, **characterized in that** the collimator opening (79) of the selective collimator (75) is variable.

18. Arrangement according to one of the preceding Claims 12 to 17, **characterized in that** the selective collimator (75) is designed as a multileaf collimator.

## Revendications

1. Dispositif pour générer un faisceau d'ions pour la thérapie par rayonnement ionique, comprenant un laser, servant à générer un faisceau laser (5), et une cible (7), le faisceau laser (5) atteignant la cible (7) de façon à générer un faisceau d'ions (9) à grande dispersion et à large spectre énergétique, comprenant un premier collimateur (11) disposé après la cible (7) et pourvu d'une ouverture de collimateur (13), à travers laquelle passe une partie du faisceau d'ions (9) à grande dispersion, de façon à produire un faisceau collimaté (15), et un dispositif de filtrage énergétique (1) comportant au moins un modulateur passif (17') servant à durcir le faisceau (15), de façon que le spectre énergétique dN/dE du faisceau (15) soit modifié par le modulateur passif (17') afin que le profil énergétique du faisceau (15) soit décalé vers des énergies plus élevées, le modulateur (17') comportant ce qui suit : un filtre magnétique (43) pour générer un premier champ magnétique, de façon à produire un faisceau à étalement spectral (47) qui est séparé spatialement suivant l'énergie des particules, un absorbeur (45) servant à homogénéiser l'énergie du faisceau étalé (47), et un second collimateur (51) par lequel sont définies une limite énergétique supérieure et une limite énergétique inférieure du faisceau étalé (47) qui est passé à travers l'absorbeur (45).

2. Dispositif selon la revendication 1, **caractérisé en**

**ce que** le filtre (43) est conçu de façon que le premier champ magnétique soit un champ magnétique homogène.

3. Dispositif selon une des revendications précédentes, **caractérisé en ce que** le filtre magnétique (43) est réalisé par un dipôle électromagnétique.

4. Dispositif selon une des revendications précédentes, **caractérisé en ce que** l'absorbeur (45) est un coin absorbant.

5. Dispositif selon une des revendications précédentes, **caractérisé en ce que** l'absorbeur (45) présente une épaisseur non homogène et est conformé en coin.

6. Dispositif selon une des revendications précédentes, **caractérisé en ce que** les parois (49a, 49b) de l'absorbeur (45), qui forment un angle aigu, s'étendent en ligne droite.

7. Dispositif selon une des revendications précédentes 1 à 5, **caractérisé en ce que** les parois (49a, 49b) de l'absorbeur (45), qui forment un angle aigu, présentent une courbure concave ou convexe.

8. Dispositif selon une des revendications précédentes, **caractérisé en ce que** l'absorbeur (45) est disposé sur le parcours des rayons du faisceau (47) étalé spectralement par le filtre magnétique (43), de façon que les particules de plus haute énergie passent par une région plus épaisse que les particules de plus faible énergie.

9. Dispositif selon une des revendications précédentes, **caractérisé par** un aimant (55) pour générer un second champ magnétique servant à paralléliser le faisceau étalé.

10. Dispositif selon la revendication précédente, **caractérisé en ce que** le second champ magnétique de l'aimant (55) est polarisé en sens inverse du premier champ magnétique du filtre magnétique (43).

11. Dispositif selon une des deux revendications précédentes, **caractérisé en ce que** le second collimateur (51) est prévu avant le second champ magnétique de l'aimant (55) par rapport à la direction du faisceau.

12. Dispositif pour générer un faisceau d'ions pour la thérapie par rayonnement ionique, comprenant un laser, servant à générer un faisceau laser, (5) et une cible (7), le faisceau laser (5) atteignant la cible (7), de façon à générer un faisceau d'ions (9) à grande dispersion et à large spectre énergétique, comprenant un premier collimateur (11) disposé après la cible (7) et pourvu d'une ouverture de collimateur (13), à travers laquelle passe une partie du faisceau d'ions (9) à grande dispersion, de façon à produire un faisceau collimaté (15), et un dispositif de filtrage énergétique (1) comportant au moins un modulateur passif (17") servant à durcir le faisceau (15), de façon que le spectre énergétique dN/dE du faisceau (15) soit modifié par le modulateur passif (17") afin que le profil énergétique du faisceau (15) soit décalé vers des énergies plus élevées, le modulateur (17") comportant un filtre non linéaire qui comprend un premier filtre magnétique (59) pour séparer spatialement le faisceau suivant l'énergie des particules, lequel présente un premier champ magnétique, et un dispositif (61) pour couper l'intensité des différentes énergies, de façon que les composantes de faisceau séparées suivant l'énergie des particules soient coupées plus ou moins fortement, le dispositif pour couper l'intensité des différentes énergies comportant un collimateur sélectif (75) avec une ouverture de collimateur rétrécie (79), et l'ouverture de collimateur rétrécie (79) du collimateur sélectif (75) possédant une largeur de passage supérieure plus grande pour les composantes de faisceau de plus haute énergie faiblement déviées que pour les composantes de faisceau de faible énergie plus fortement déviées.

13. Dispositif selon la revendication 12, **caractérisé en ce que** le premier champ magnétique est un champ magnétique homogène.

14. Dispositif selon une des revendications précédentes 12 à 13, **caractérisé en ce que** le modulateur (17") comporte un aimant (71) avec un second champ magnétique qui est polarisé en sens inverse du premier champ magnétique du filtre magnétique (59).

15. Dispositif selon la revendication précédente 14, **caractérisé en ce que** le second champ magnétique est un champ magnétique homogène.

16. Dispositif selon une des revendications précédentes 14 à 15, **caractérisé en ce qu'**un second collimateur (65) est placé avant l'aimant (71) avec le second champ magnétique.

17. Dispositif selon une des revendications précédentes 12 à 16, **caractérisé en ce que** l'ouverture de collimateur (79) du collimateur sélectif (75) est variable.

18. Dispositif selon une des revendications précédentes 12 à 17, **caractérisé en ce que** le collimateur sélectif (75) est conçu comme un collimateur multilame.

Fig.1

Fig.1a

Fig.1b

EP 1 482 519 B1

Fig.2

Fig.2a

Fig.2b

Fig.3

Fig.3a

Fig.3b

Fig.4

EP 1 482 519 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 0049624 A
- JP 06154351 A
- US 5440133 A

### In der Beschreibung aufgeführte Nicht-Patentliteratur

- Optic of charged particles. **Hermann Wollnik.** Academic Express. 253-277
- **Fourkal et al.** Particle in cell simulation of laser-accelerated proton beams for radiation therapy. *Med. Phys.,* Dezember 2002, vol. 29 (12), 2788-2798
- **von W.P. Jones et al.** Design of a beam transport system for proton radiation therapy facility. *Proceedings of the 1999 Particle Accelerator Conference,* 1999, 2519-2521